# EUROPEAN PATENT APPLICATION

(11) **EP 4 332 234 A1**
(43) Date of publication of application: **06.03.2024**
(21) Application number: 22192680.1
(22) Date of filing: 29.08.2022
(51) Int. Cl.: C12Q 1/6806, C12Q 1/6804

(54) **IMPROVED MICROFLUIDIC TRANSCRIPTOME BASED SCREENING METHODS**

(71) Applicant: ECOLE POLYTECHNIQUE FEDERALE DE LAUSANNE (EPFL), 1015 Lausanne (CH)
(72) Inventor: Hu, Hongxing, 1015 Lausanne (CH); Merten, Christoph, 1015 Lausanne (CH); Ma, Xiaoli, 1015 Lausanne (CH); Kolmar, Leonie, 1015 Lausanne (CH)
(74) Representative: Kuttenkeuler, David

(57) **Abstract**

The invention provides microfluidic screening systems for the identification of compounds or compositions influencing cellular transcriptomes. The invention is predicated upon taking into account differences in newly synthesized mRNA in contrast to so called old mRNA and such differences compared to control cell assays. The invention is applicable for screening in particular candidate ligand molecules such as antibodies for an effect on target cells, and thereby provides new strategies for the identification of therapeutically active compounds.

## Description

### FIELD OF THE INVENTION

The invention provides (micro)fluidic screening systems for the identification of compounds or compositions influencing cellular transcriptomes. The invention is predicated upon taking into account differences in newly synthesized mRNA in contrast to so called old mRNA and such differences compared to control cell assays. The invention is applicable for screening in particular candidate ligand molecules such as antibodies for an effect on target cells, and thereby provides new strategies for the identification of therapeutically active compounds.

### DESCRIPTION

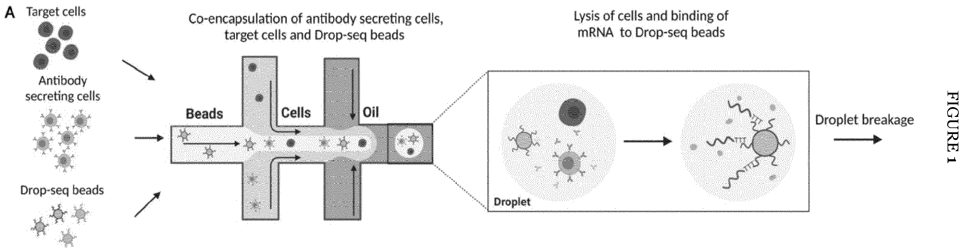

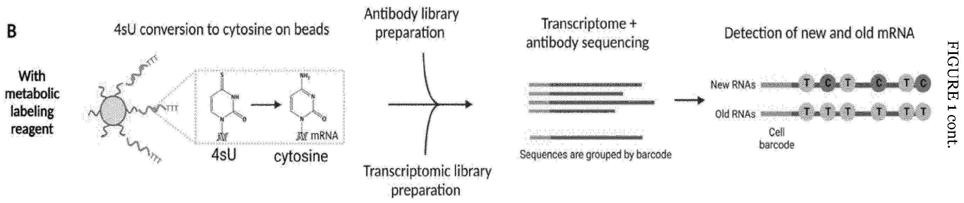

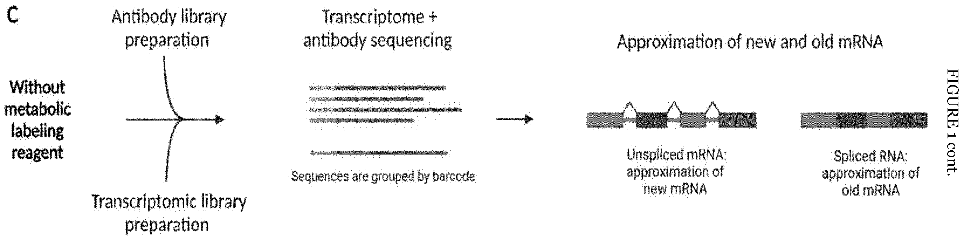

Compartment based (micro)fluidics, such as droplet based microfluidics, holds great potential for high throughput screening applications. The encapsulation of single cells into compartments allows screening of cell products such as antibodies at very high throughput, e.g. up to several hundred thousand samples per day. Single cell RNA sequencing, also termed RNAseq, has gained a lot of scientific and commercial attention. Its goal is the analysis of global gene expression patterns, or targeted analysis of gene subsets, on the single-cell level, thus allowing to analyse and reveal distinct cell types in heterogeneous populations such as stem cells, tumours or developing embryos. Fluidigm Corp. has become the market leader of this technology offering valve -based microfluidic solutions for single cell RNAseq. Their CI system can process up to 96 cells in a single run and the next generation of this platform will have a throughput of up to 800 cells in one go. In parallel, nanowells (Fan, H.C., G.K. Fu, and S.P.A. Fodor, Science, 2015. 347(6222): p. 628) and droplet based microfluidic technology (Macosko, E.Z. et al., Cell, 2015. 161(5): p. 1202-14; Klein, A.M., et al., Cell, 2015. 161(5): p. 1187-201; Rotem, A., et al., Nat Biotechnol, 2015. 33(11): p. 1165-72) have been developed, enabling the processing of up to 10,000 cells for RNAseq or ChiPseq. In these systems, single-cells are encapsulated into microfluidic droplets or nanowells, together with single beads displaying polyT nucleotides with unique barcodes (Fig. 1). After encapsulation the cells are lysed and all cellular mRNAs hybridize with the barcoded polyT primers, thus ensuring a physical linkage with the barcode. Either at this stage or after performing an additional reverse transcription step within the droplets all samples can be pooled and applied to next generation sequencing. Due to the barcoding the expression patterns of individual cells can still be distinguished, thus revealing differences within the population. Prior art single cell sequencing techniques and studies focused originally on characterizing single cells individually. However, newer methods such as CIMseq or ProximID technologies rely on partial disruption of tissue, and they use high-throughput transcriptomic analysis of the multiplets to explore the interacting partner cells. Similarly, PIC-seq makes use of fluorescence-activated cell sorting to identify multiplets of physically interacting cells (e.g., duplets of T cells and dendritic cells) that are sorted into wells of a microtiter plate for downstream RNA sequencing analysis. Such a co -encapsulation of two different cell types into the same droplet and labeling the mRNAs of both cell types with the same barcodes has huge biomedical potential: It allows analyzing cell-cell interactions, e.g. how cell A reacts to the presence of cell B or factors secreted by cell B, and could in particular be exploited for screening genetically-encoded drug candidates such as monoclonal antibodies, which have annual sales of more than 50 billion US$, in a highly multiplexed fashion.

WO/2017/121832 discloses a transcriptome-based screening approach using a barcoding system for RNA molecules in each compartment of the microfluidic approach. Exemplary approaches include the immunization of animals with human cancer cells or membrane extracts thereof. Subsequently, plasma cells secreting antibodies against cell epitopes and receptors can be isolated and applied to screens, based on the co-compartmentalization of a single plasma cell and a single cancer cell into compartments, together with a single bead displaying polyT primers harboring unique barcodes (a different barcode for each bead and hence for each compartment). Subsequent to the generation of compartments hosting plasma and cancer cells, the samples can be incubated to allow for efficient secretion of antibodies acting on the target cell. It is well known that antibodies cannot only bind to surface receptors, but as well trigger signal cascades ultimately resulting in changed expression profiles (Silva, H.M., et al., Immunol Lett, 2009.125(2): p. 129-36; Franke, A., et al., PLoS One, 2011. 6(2): p. e 1 6596). After an incubation period, the plasma cell and the cancer cell can be lysed inside the compartments, and the cellular mRNAs hybridize with the barcoded polyT primer in the compartments. This ensures that the mRNAs of both cell types in each compartment is physically linked to/labeled with the same barcode. In other words, both the antibody encoding genes of the animal plasma cell as well as the genes expressed in the human cancer cell upon contact with the animal antibodies get labelled with the same barcode. Either at this step or after first strand cDNA synthesis inside the compartments, the contents of all compartments are pooled. Subsequently a next generation sequencing library is generated and sequenced. Then, the resulting data is analyzed based on the barcodes revealing both the identity of the antibody that was present in a particular compartment as well as its effect on the expression pattern of the human cell.

However, the approach disclosed in WO/2017/121832 was shown to be able to identify highly significant transcriptional changes, in particular in scenarios where multiple cells are used. Sensitivity of the detection of a more comprehensive transcriptional change in the screening compartments is however still a challenge due to a reduced clustering of screening hits in the subsequent RNA sequencing analysis.

Thus, it is an object of the invention to provide an improved (micro)fluidic based transcriptome screening system for the identification of potentially therapeutic compounds and compositions.

### BRIEF DESCRIPTION OF THE INVENTION

Generally, and by way of brief description, the main aspects solving the problem of the present invention can be described as follows:

In **a first aspect,** the invention pertains to a method for screening in a fluidic, such as microfluidic, system candidate compounds or compositions for an effect on a cell, the method comprising the steps of
(a) Providing a candidate compound or composition and a first cell, wherein the candidate compound or composition is screened for an effect on the first cell;
(b) Encapsulating a candidate compound or composition together with a first cell into a fluidic, preferably microfluidic, compartment and thereby bringing into contact the candidate compound or composition and the first cell;
(c) Incubating the fluidic, preferably microfluidic, compartment for a time sufficient to allow the first cell to change its transcriptome in response to contacting the candidate compound or composition;
(d) Determining by sequencing a transcriptome of the first cell, wherein the determining the transcriptome of the first cell comprises determining a new transcriptome of the first cell which comprises newly synthesized mRNA expressed in the first cell after a predetermined point in time, such as the encapsulation in step (b) and an old transcriptome of the first cell which comprises synthesized mRNA expressed and present before the predetermined point in time, such as the encapsulation in step (b);
(e) Wherein a change of mRNA expression in the new transcriptome compared to the old transcriptome in the first cell indicates an effect of the candidate compound or composition on the first cell.

In **a first alternate aspect,** the invention pertains to a method for screening in a (micro)fluidic system candidate compounds or compositions for an effect on a cell, the method comprising the steps of
(a) Providing a candidate compound or composition and a first cell, wherein the candidate compound or composition is screened for an effect on the first cell;
(b) Encapsulating a candidate compound or composition together with a first cell into a fluidic, preferably microfluidic, compartment and thereby bringing into contact the candidate compound or composition and the first cell; wherein the fluidic, preferably microfluidic, compartment further comprises a label-agent for selectively labeling synthesized mRNA in the first cell;
(c) Incubating the fluidic, preferably microfluidic, compartment for a time sufficient to allow the first cell to change its transcriptome in response to contacting the candidate compound or composition;
(d) Determining by sequencing the transcriptome of the first cell, wherein the transcriptome is composed of a new transcriptome comprising newly synthesized mRNA detectable by the label-agent after encapsulation in step (b) and an old transcriptome comprising synthesized mRNA detectable by the absence of the label-agent before encapsulation in step (b);
Wherein a change of mRNA expression in the new transcriptome compared to the old transcriptome in the first cell indicates an effect of the candidate compound or composition on the first cell.

In **a second aspect,** the invention pertains to a plurality of fluidic, preferably microfluidic, compartments, wherein at least 1% of said compartments form a subset in which each compartment comprises
(a) A first cell;
(b) A candidate compound or composition;
(c) A label-agent for selectively labeling synthesized mRNA in the first cell; and
(d) Optionally, a set of barcode oligonucleotides each comprising a barcode sequence unique to the set and a sequence capable of binding specifically to mRNA and/or cDNA.

In **a third aspect,** the invention pertains to a method for generating a plurality of fluidic, preferably microfluidic, compartments according to the second aspect, comprising the steps of:
(a) introducing into a (micro)fluidic system: (i) a fluid comprising a plurality of first cells, (ii) a fluid comprising a plurality of candidate compounds or compositions, (iii) a fluid comprising a label-agent for selectively labeling synthesized mRNA in the first cell; and (iv) a fluid comprising sets of barcode oligonucleotides, wherein the barcode oligonucleotides of each set comprises a barcode sequence unique to the set, and a sequence capable of binding specifically to mRNA and/or cDNA, and
(b) repeatedly co-compartmentalizing a first cell, a candidate compound or composition, a label-agent for selectively labelling synthesized mRNA, and a set of barcode oligonucleotides into fluidic, preferably microfluidic, compartments, such that the size of the subset of compartments in the plurality of compartments is at least 1%.

In **a fourth aspect,** the invention pertains to a method for determining a differential gene expression of a cell, comprising the steps:
(a) Providing a first cell exposed to a stimulus, such as a candidate compound or composition, wherein the candidate compound or composition is screened for an stimulus (effect) on the first cell;
(b) Encapsulating the first cell and, optionally the compound or composition, into a fluidic, preferably microfluidic, compartment and, optionally, thereby bringing into contact the candidate compound or composition and the first cell;
(c) Incubating the fluidic, preferably microfluidic, compartment for a time sufficient to allow the first cell to change its transcriptome in response to the stimulus, such as contacting the candidate compound or composition;
(d) lysing the first cell comprised in the fluidic, preferably microfluidic, compartment,
(e) Determining by sequencing a transcriptome of the first cell, wherein the determining the transcriptome of the first cell comprises determining a new transcriptome of the first cell which comprises newly synthesized mRNA expressed in the first cell after encapsulation in step (b) and an old transcriptome of the first cell which comprises synthesized mRNA expressed and present before encapsulation in step (b);
(f) Providing sequencing data of a control new transcriptome (pre-identified) of one or more control cell(s) not contacted with the stimulus, such as the candidate compound or composition, wherein the first cell and the one or more control cell(s) are genetically clonal and comprise the same purposefully introduced genetic modifications;
(g) comparing the new transcriptome of first cell with the control new transcriptome wherein a change in the new transcriptome of the first cell compared to the control new transcriptome indicates an effect of the stimulus, such as a compound or composition, on the first cell.

In **a fourth alternate aspect,** the invention pertains to a method for determining a differential gene expression of a cell, comprising the steps:
(a) Providing a first cell exposed to a stimulus, such as a candidate compound or composition, wherein the candidate compound or composition is screened for an stimulus (effect) on the first cell;
(b) Encapsulating the first cell and, optionally the compound or composition, into a fluidic, preferably microfluidic, compartment and, optionally, thereby bringing into contact the candidate compound or composition and the first cell; wherein the fluidic, preferably microfluidic, compartment further comprises a label-agent for selectively labeling (newly) synthesized mRNA in the first cell;
(c) Incubating the fluidic, preferably microfluidic, compartment for a time sufficient to allow the first cell to change its transcriptome in response to the stimulus, such as contacting the candidate compound or composition;
(d) lysing the first cell comprised in the fluidic, preferably microfluidic, compartment,
(e) Determining by sequencing the transcriptome of the first cell, wherein the transcriptome of the first cell is composed of a new transcriptome comprising newly synthesized mRNA detectable by the label-agent after encapsulation in step (b), and an old transcriptome comprising previously synthesized mRNA detectable by the absence of the label-agent before encapsulation in step (b);
(f) Providing sequencing data of a control new transcriptome (pre-identified) of one or more control cell(s) not contacted with the stimulus, such as the candidate compound or composition, wherein the first cell and the one or more control cell(s) are genetically clonal and comprise the same purposefully introduced genetic modifications;
(g) comparing the new transcriptome of first cell with the control new transcriptome wherein a change in the new transcriptome of the first cell compared to the control new transcriptome indicates an effect of the stimulus, such as a compound or composition, on the first cell.

### DETAILED DESCRIPTION OF THE INVENTION

In the following, the elements of the invention will be described. These elements are listed with specific embodiments, however, it should be understood that they may be combined in any manner and in any number to create additional embodiments. The variously described examples and preferred embodiments should not be construed to limit the present invention to only the explicitly described embodiments. This description should be understood to support and encompass embodiments which combine two or more of the explicitly described embodiments or which combine the one or more of the explicitly described embodiments with any number of the disclosed and/or preferred elements. Furthermore, any permutations and combinations of all described elements in this application should be considered disclosed by the description of the present application unless the context indicates otherwise.

In **a first aspect,** the invention pertains to a method for screening in a fluidic, such as microfluidic, system candidate compounds or compositions for an effect on a cell, the method comprising the steps of
(a) Providing a candidate compound or composition and a first cell, wherein the candidate compound or composition is screened for an effect on the first cell;
(b) Encapsulating a candidate compound or composition together with a first cell into a fluidic, preferably microfluidic, compartment and thereby bringing into contact the candidate compound or composition and the first cell;
(c) Incubating the fluidic, preferably microfluidic, compartment for a time sufficient to allow the first cell to change its transcriptome in response to contacting the candidate compound or composition;
(d) Determining by sequencing a transcriptome of the first cell, wherein the determining the transcriptome of the first cell comprises determining a new transcriptome of the first cell which comprises newly synthesized mRNA expressed in the first cell after a predetermined point in time, such as the encapsulation in step (b) and an old transcriptome of the first cell which comprises synthesized mRNA expressed and present before the predetermined point in time, such as the encapsulation in step (b);
(e) Wherein a change of mRNA expression in the new transcriptome compared to the old transcriptome in the first cell indicates an effect of the candidate compound or composition on the first cell.

In **a first alternate aspect,** the invention pertains to a method for screening in a microfluidic system candidate compounds or compositions for an effect on a cell, the method comprising the steps of
(a) Providing a candidate compound or composition and a first cell, wherein the candidate compound or composition is screened for an effect on the first cell;
(b) Encapsulating a candidate compound or composition together with a first cell into a fluidic, preferably microfluidic, compartment and thereby bringing into contact the candidate compound or composition and the first cell; wherein the fluidic, preferably microfluidic, compartment further comprises a label-agent for selectively labeling synthesized mRNA in the first cell;
(c) Incubating the fluidic, preferably microfluidic, compartment for a time sufficient to allow the first cell to change its transcriptome in response to contacting the candidate compound or composition;
(d) Determining by sequencing the transcriptome of the first cell, wherein the transcriptome is composed of a new transcriptome comprising newly synthesized mRNA detectable by the label-agent after encapsulation in step (b) and an old transcriptome comprising synthesized mRNA detectable by the absence of the label-agent before encapsulation in step (b);
Wherein a change of mRNA expression in the new transcriptome compared to the old transcriptome in the first cell indicates an effect of the candidate compound or composition on the first cell.

8abellingThe present invention provides an improved transcriptome-based microfluidic screening approach by taking into account the difference between (x) newly synthesized mRNA in a cell upon a stimulus and (y) newly synthesized mRNA in a control cell not being exposed to the same stimulus. In context of the invention the point in time selected for differentiation is preferably defined by the exposure to the stimulus. In addition, the method of the invention can differentiate between mRNA newly synthesized (new mRNA or "new transcriptome"), preferably synthesized upon and after stimulus exposure and mRNA synthesized in the same cell before stimulus exposure (old mRNA or "old transcriptome"). By using the mentioned control mechanisms, the invention provides an improved protocol for the identification of the transcriptional changes, including absolute and differential quantity of transcribed RNA change and RNA identity, upon possibly any controllable stimulus that may be subject of a screening investigation. In preferred embodiment, the control cell population for defining a change between new and old transcriptomes may also be derived from a larger test population of cells which comprises only a smaller fraction of cells wherein a predetermined threshold of differential expression is observed.

Therefore, one feature of the invention is based on the labelling of newly synthesized mRNA opposed to "old" mRNA, which can be done for example by adding to the cells a chemical compound that labels mRNA during the process of transcription, but not already transcribed mRNA. Such labels then need to be detectable in a later sequencing analysis One option for such an approach is disclosed by Qiu et al 2020 (Nature Methods volume 17, pages 991-1001; 2020), who have described previously a method in which newly synthesized mRNA is chemically labelled with a reagent termed 4-thiouridine (4sU) which is immediately taken up by cells, phosphorylated, and incorporated into any newly transcribed RNA (mRNA and any other transcribed RNA). The incorporated 4sU is used to induce C->T conversions in the sequencing library upon additional treatment with the reagent "TEFA". They show that applying the labelling of newly synthesized RNA together with a particular treatment or stimulus allows to distinguish treated versus untreated cells (based on the transcriptome) at much better resolution.

The step of determining the transcriptome of the first cell in accordance with the invention, preferably in step (d) of the method of the first aspect, further comprises a comparison of the new transcriptome of first cell with a pre-identified new transcriptome of a one or more control cell(s) not contacted a stimulus, such as with any candidate compound or composition, and wherein a change in the new transcriptome of the first cell compared to the new transcriptome of the one or more control cell(s) indicates an effect of the stimulus, such as the compound or composition on the first cell; wherein the first cell and the one or more control cell(s) are genetically clonal and comprise the same purposefully introduced genetic modifications.

The point of time that is defined to differentiate between an old and a new transcriptome in context of the invention may be selected depending on the application of the method of invention. Usually such a point in time is selected to be when a cell is brought into contact with a certain stimulus which is subject to investigation in an experiment or screening. As such a preferred point in time is the time when the stimulus and the first cell are in contact, or capable of getting into contact (directly or indirectly) for the first time, such as when both are encapsulated in the same compartment.

As a further preferred embodiment of the method of the first aspect, the candidate compound or composition has an effect on the first cell if the number of differentially expressed mRNA in the new transcriptome of the first cell compared to the new transcriptome of the one or more control cell(s) divided by the number of total expressed RNA species (such as mRNA, and therefore expressed genes) in the old transcriptome of the first cell is higher than a predetermined threshold P.

The term "transcriptome," as used herein, is defined as the set of all or a part of, RNA molecules expressed in one cell or a population of cells. A transcriptome can denote the complete and detectable set of RNAs, or can be specifically denote a subset of RNA molecules of interest. As such a transcriptome may comprise a transcriptome subset of a selected number of expressed RNAs, such as mRNA from a selection of genes (2, 3, 4, 5 or more, 10 or more, 100 or more, 1000, 10000 or more), or may be a subset of types of RNA, such as messenger RNA, structural RNA, or any other subset of RNA molecules expressed. The set of RNA molecules may include messenger RNAs and/or microRNAs and other small RNAs. The term may refer to the total set of RNA molecules in a given organism, or to the specific subset of RNA molecules present in a particular cell type. A preferred embodiment of the invention pertains to mRNA as species of RNA expressed and investigated in accordance with the invention.

The term "new transcriptome" in accordance with the invention pertains to the all RNA molecules expressed in one cell or a population of cells which are transcribed upon and after exposing the cell or cells to a stimulus, such as a compound or composition, which is investigated for an effect on the transcriptome of the cell or population of cells (in the method of the invention referred to as the first cell). Therefore, the term "old transcriptome" pertains toall RNA molecules expressed in one cell or a population of cells which were transcribed before, and up until exposing the cell or cells to a stimulus, such as a compound or composition.

In one embodiment of the invention the method further comprises that determining the old or new transcriptome of the more than one control cells involves calculating the average of the transcriptomes (both old and new transcriptomes) of the more than one control cells.

The term "fluidic compartment", or "microfluidic compartment" or "microcompartment" as used herein refers to a compartment of a certain size that comprises or encapsulates an aqueous liquid. The size of the microfluidic compartment is usually less than 1 microlitre (µl). Preferably, it is less than 1,000 nl, less than 100 nl, less than 20 nl, or most preferably less than 1 nl. The lower size limit is 1 pl, preferably 10 pl. The compartments can be provided as droplets or wells or any other structure able to provide multiple aqueous compartments.

A wide variety of compartmentalisation or microencapsulation procedures are available (Benita, S., Ed. (1996). Microencapsulation: methods and industrial applications. Drugs and pharmaceutical sciences. Edited by Swarbrick, J. New York: Marcel Dekker) and may be used to create the (micro)fluidic compartment used in accordance with the present invention. Indeed, more than 200 microencapsulation or compartmentalisation methods have been identified in the literature (Finch, C. A. (1993) Encapsulation and controlled release. Spec. Publ.-R. Soc. Chem. 138, 35). These include membrane enveloped aqueous vesicles such as lipid vesicles (liposomes) (New, R. R. C, Ed. (1990). Liposomes: a practical approach. The practical approach series. Edited by Rickwood, D. & Hames, B. D. Oxford: Oxford University Press) and non-ionic surfactant vesicles (van Hal, D. A., Bouwstra, J. A. & Junginger, H. E. (1996). Nonionic surfactant vesicles containing estradiol for topical application. In Microencapsulation: methods and industrial applications (Benita, S., ed.), pp. 329-347. Marcel Dekker, New York.). In a preferred embodiment, the (micro)fluidic compartment is selected from the group consisting of droplets, nanowells and valve -based (micro)fluidic compartments. A nanowell is a cavity of any shape and depth. Preferably, it has a diameter of less than two-fold the diameter of a solid particle as defined below so that only individual beads get trapped in the compartment (Fan HC, Fu GK, Fodor SP. Science. 347(6222): 1258367, 2015. doi: 10.1126/science.1258367). In other words, the diameter of at least 200 nm and of up to 100 µιη, preferably of 600 nm to 20 µιη. The volume of a nanowell preferably is 1 pL to 10 nL. A valve-based compartment is a section of a (micro)fluidic channel that is closed-off or pinched-off by (micro)fluidic valves (Thorsen T, Maerkl SJ, Quake SR, Science 298(5593): 580-584, 2002. DOI: 10.1126/science.1076996). The volume of a valve-based compartments preferably is 1 pL to 10 nL Preferably, the (micro)fluidic compartment is a (micro)fluidic droplet of an aqueous liquid in an immiscible liquid. Thus, preferably the microcompartments of the present invention are formed from emulsions; heterogeneous systems of two immiscible liquid phases with one of the phases dispersed in the other as droplets of microscopic size (Becher, P. (1957) Emulsions: theory and practice. Reinhold, New York; Sherman, P. (1968) Emulsion science. Academic Press, London; Lissant, K.J., ed Emulsions and emulsion technology. Surfactant Science New York: Marcel Dekker, 1974; Lissant, K.J., ed. Emulsions and emulsion technology. Surfactant Science New York: Marcel Dekker, 1984). Emulsions may be produced from any suitable combination of immiscible liquids. Preferably the emulsion of the present invention has water (containing a particle and other components) as the phase present in the form of droplets and a hydrophobic, immiscible liquid (preferably an oil) as the surrounding matrix in which these droplets are suspended. Such emulsions are termed 'water-in-oil'. This has the advantage that the aqueous phase is compartmentalised in discrete droplets. The external phase, preferably being a hydrophobic oil, generally is inert. The emulsion may be stabilized by addition of one or more surface-active agents (surfactants). These surfactants act at the water/oil interface to prevent (or at least delay) separation of the phases. Many oils and many emulsifiers can be used for the generation of water-in-oil emulsions; a recent compilation listed over 16,000 surfactants, many of which are used as emulsifying agents (Ash, M. and Ash,I. (1993) Handbook of industrial surfactants. Gower, Aldershot).

Preferably, the aqueous microcompartments are created, handled and/or controlled in a (micro)fluidic system. This technology is based on the manipulation of continuous liquid flow through microfabricated channels. Actuation of liquid flow is implemented either by external pressure sources, external mechanical pumps, integrated mechanical micropumps, or by combinations of capillary forces and electrokinetic mechanisms. Process monitoring capabilities in continuous-flow systems can be achieved with highly sensitive (micro)fluidic flow sensors based on MEMS technology which offer resolutions down to the nanoliter range.

(micro)fluidic devices typically consist of networks of channels of approximately ten to a few hundred micrometers in diameter into which small quantities of reagents can be injected in a specific sequence, mixed and incubated for a specified time. Assays can be highly parallelized by generating independent compartments using valves (pinching off specific regions of the channels) or two-phase (micro)fluidics, in which aqueous droplets surrounded by an immiscible oil phase serve as closed vessels. These approaches enable drastically reduced assay volumes (pico -nanoliters) and strongly improved throughput. For example, compartments can be generated at rates of more than 1,000 per second. Furthermore, (micro)fluidic modules for the splitting, fusion and sorting of compartments at similar rates have been developed, thus providing a repertoire of manipulations mimicking classical bench top procedures.

In a preferred embodiment (applying to all aspects herein), the device, in particular the channels, is/are large enough to handle compartments comprising eukaryotic cells. In other words, the device, in particular the channels, is/are large enough to handle compartments of the sizes described herein, in particular 660 pl droplets.

The term "first cell" as used herein refers to any cell, preferably it is a mammalian cell and more preferably a human cell. In one embodiment, the cell is a stem or pluripotent cell, e.g. an embryonic or adult stem cell, or a non-stem and non-pluripotent cell, preferably in which pluripotency is inducible by a polypeptide ligand. Such a non-stem cell and non-pluripotent cell is preferably a cell derived from the ectoderm, endoderm or mesoderm lineage. Said cell can be selected from the group consisting of growth-arrested cells (e.g. cell which are blocked at various stages of the cell cycle, i.e. GO, Gl, S, G2, prophase, prometaphase and metaphase), non-proliferating cells, post- or non-mitotic cells, resting cells, benign cells, senescent cells, in vitro differentiated embryonic stem cells, in vitro differentiated induced pluripotent cells, terminally differentiated cells, and preferably primary cells. Preferred cells are cells selected from the group consisting of adipocytes, astrocytes, B-cells, cardiomyocytes, chondrocytes, cornea epithelial cells, dendritic cells, endocrine cells, endothelial cells, epithelial cells, fibroblasts, glia cells, granulocytes, hematopoietic cells, hematopoietic stem cells, hepatocytes, keratinocytes, intestinal epithelial cells, liver cells, lung epithelial cells type I, lung epithelial cells type II, lymphocytes, macrophages, mammary epithelial cells, melanocytes, mesangial cells, mesenchymal stem cells, muscle cells, myoblast, natural killer cells, neuronal cells, neutrophiles, osteoblasts, pancreatic beta cells, pericytes, preadipocytes, progenitor cells, prostate epithelial cells, renal epithelial cells, renal proximal tubule cells, retinal pigment epithelial cells, Sertoli cells, skeletal muscle cells, smooth muscle cells, stem cells, stroma cells, T-cells and subsets of said cell types. Said cells are non-mammalian cells (e.g. from fish or bird species) or mammalian cells (e.g. from mice, rats, monkeys, pigs, dogs, cats, cows, sheep, goats), preferably human cells.

In a preferred embodiment, the first cell is a diseased cell. In particular, the diseased cell may be a tumor cell, a chronically infected cell, a senescent cell, a cell showing an inflammatory phenotype, a cell accumulating amyloid proteins or a cell accumulating misfolded proteins.

In case of a tumor cell, the underlying disease is a tumor, preferably selected from the group consisting of Adrenal Cancer, Anal Cancer, Bile Duct Cancer, Bladder Cancer, Bone Cancer, Brain/CNS, Tumors, Breast Cancer, Cancer of Unknown Primary, Castleman Disease, Cervical Cancer, Colon/Rectum Cancer, Endometrial Cancer, Esophagus Cancer, Ewing Family Of Tumors, Eye Cancer, Gallbladder Cancer, Gastrointestinal Carcinoid Tumors, Gastrointestinal Stromal Tumor (GIST), Gestational Trophoblastic Disease, Hodgkin Disease, Kaposi Sarcoma, Kidney Cancer, Laryngeal and Hypopharyngeal Cancer, Leukemia, Liver Cancer, Lung Cancer, Lymphoma, Lymphoma of the Skin, Malignant Mesothelioma, Multiple Myeloma, Myelodysplasia Syndrome, Nasal Cavity and Paranasal Sinus Cancer, Nasopharyngeal Cancer, Neuroblastoma, Non-Hodgkin Lymphoma, Oral Cavity and Oropharyngeal Cancer, Osteosarcoma, Ovarian Cancer, Pancreatic Cancer, Penile Cancer, Pituitary Tumors, Prostate Cancer, Retinoblastoma, Rhabdomyosarcoma, Salivary Gland Cancer, Sarcoma - Adult Soft Tissue Cancer, Skin Cancer, Small Intestine Cancer, Stomach Cancer, Testicular Cancer, Thymus Cancer, Thyroid Cancer, Uterine Sarcoma, Vaginal Cancer, Vulvar Cancer, Waldnstrom Macroglobulinemia, and Wilms Tumor.

In case of a chronically infected cell, the underlying disease is a chronic infectious disease, such as tuberculosis, malaria, chronic viral hepatitis (HBV, Hepatitis D virus and HCV), Acquired immune deficiency syndrome (AIDS, caused by HIV, Human Immunodeficiency Virus), or EBV related disorders: Systemic Autoimmune Diseases (Systemic Lupus Erithematosus, Rheumatoid Arthritis, and Sjogren Syndrome) and Multiple Sclerosis (MS). Preferably, the chronically infected cell comprises a pathogen or part thereof of the above-recited infectious diseases.

In case of a senescent cell, the underlying disease is a senescence associated disease, such as (i) rare genetic diseases called Progeroid syndromes, characterized by pre-mature aging: Werner syndrome (WS), Bloom syndrome (BS), Rothmund-Thomson syndrome (RTS), Cockayne syndrome (CS), Xeroderma pigmentosum (XP), Trichothiodystrophy or Hutchinson-Gilford Progeria Syndrome (HGPS) or (ii) Common age related disorders: Obesity, type 2 diabetes, sarcopenia, osteoarthritis, idiopathic pulmonary fibrosis and chronic obstructive pulmonary disease, cataracts, neurodegenerative diseases, or cancer treatment related disorders.

Preferably, the senescent cell expresses, in particular in a misfolded form and/or presented on the cell surface, one or more protein such as prion protein (PrP), FasR, Fas ligand, CD44, EGF receptor, CD38, Notch- 1, CD44, CD59, or TNF receptor. Notwithstanding, the first cell may also be a non-diseased cell expressing one or more of these proteins.

In case of a cell showing an inflammatory phenotype, the underlying disease is an inflammatory disease, such as an Allergy, Asthma, Artherosclerosis, Autoimmune diseases, Autoinflammatory diseases, Celiac disease, Chronic prostatitis, Glomerulonephritis, Hypersensitivities, Inflammatory Bowel disease, Inflammatory myopathies, Obesity, Pelvic inflammatory disease, Reperfusion injury, Rheumatoid arthritis, Sarcoidosis, Transplant rejection, Vasculitis, or Interstitial cystitis. Preferably, a cell showing an inflammatory phenotype is a cell overexpressing one or more proinflammatory factors such as Bradykinin, C3, C5a, Factor XII, Membrance attack complex, Plasmin, Thrombin, Lysosome granules, Histamine, IFN-gamma, IL-8, IL-6, IL-8, IL-18, Leukotriene B4, Nitric oxide, Prostaglandins, TNF-alpha, or C-reactive Protein.

In case of a cell accumulating amyloid proteins, the underlying disease is a disease associated with the abnormal accumulation of amyloid fibrils such as Alzheimer's disease, Diabetes mellitus type 2, Parkinson's disease, Transmissible spongiform encephalopathy, Fatal familial insomnia, Huntington's disease, Medullary carcinoma of the thyroid, Cardiac arrythmias, Atherosclerosis, Rheumatoid arthritis, Aortic medial amyloid, Prolactinomas, Familial amyloid polyneuropathy, Hereditary non-neuropathic systemic amyloidosis, Dialysis related amyloidosis, Lattice corneal dystrophy, Cerebral amyloid angiopathy Cerebral amyloid angiopathy, Systemic AL amyloidosis, or Sporadic inclusion body myositis. Preferably, a cell accumulating amyloid proteins is a cell overexpressing one or more amyloids such as Beta amyloid, IAPP, Alpha-synuclein, PrPSc, Huntingtin, Calcitonin, Atrial natriuretic factor, Apolipoprotein Al, Serum amyloid A, Medin, Prolactin, Transthyretin, Lysozyme, Beta-2 microglobulin, Gelsolin, Keratoepithelin, Cystatin, Immunoglobulin light chain AL, or S-IBM.

In case of a cell accumulating misfolded proteins, the underlying disease is a proteopathy such as Alzheimer's disease, Cerebral β-amyloid angiopathy, Retinal ganglion cell degeneration in glaucoma, Prion diseases, Parkinson's disease, Tauopathies, Frontotemporal lobar degeneration, FTLD-FUS, Amyotrophic lateral sclerosis, Huntington's disease, Familial British dementia, Familial Danish dementia, Hereditary cerebral hemorrhage with amyloidosis, CADASIL, Alexander disease, Seipinopathies, Familial amyloidotic neuropathy, Senile systemic amyloidosis, AL (light chain) amyloidosis, AH (heavy chain) amyloidosis, AA (secondary) amyloidosis, Type II diabetes, Aortic medial amyloidosis, ApoAI amyloidosis, ApoAII amyloidosis, ApoAIV amyloidosis, Familial amyloidosis of the Finnish type, Lysozyme amyloidosis, Fibrinogen amyloidosis, Dialysis amyloidosis, Inclusion body myositis/myopathy, Cataracts,, Retinitis pigmentosa with rhodopsin mutations, Medullary thyroid carcinoma, Cardiac atrial amyloidosis, Pituitary prolactinoma, Hereditary lattice corneal dystrophy, Cutaneous lichen amyloidosis, Mallory bodies, Corneal lactoferrin amyloidosis, Pulmonary alveolar proteinosis, Odontogenic (Pindborg) tumor amyloid, Seminal vesicle amyloid, Cystic Fibrosis, Sickle cell disease, or Critical illness myopathy. Preferably, a cell accumulating misfolded proteins is a cell misfolding one or more proteins such as Amyloid β peptide (Aβ), Tau protein, Amyloid β peptide (Aβ), Amyloid β peptide (Aβ), Prion protein, a-Synuclein, Microtubule-associated protein tau (Tau protein), TDP-43, Fused in sarcoma (FUS) protein, Superoxide dismutase, TDP-43, FUS, Proteins with tandem glutamine expansions, ABri, ADan, Cystatin C, Notch3, Glial fibrillary acidic protein (GFAP), Seipin, Transthyretin, Serpins, Monoclonal immunoglobulin light chains, Immunoglobulin heavy chains, Amyloid A protein, Islet amyloid polypeptide (IAPP; amylin), Medin (lactadherin), Apolipoprotein AI, Apolipoprotein All, Apolipoprotein AIV, Gelsolin, Lysozyme, Fibrinogen, Beta-2 microglobulin, Amyloid β peptide (Aβ), Crystalline, Rhodopsin, Calcitonin, Atrial natriuretic factor, Prolactin, Keratoepithelin, Keratins, Keratin intermediate filament proteins, Lactoferrin, Surfactant protein C (SP-C), Odontogenic ameloblast-associated protein, Semenogelin I, cystic fibrosis transmembrane conductance regulator (CFTR) protein, Hemoglobin, or Hyperproteolytic state of myosin ubiquitination.

In context of the invention the "stimulus" that is exposed to the first cell in any of the various aspects of the invention may be any kind deliberately induced effect on or within the first cell of the invention. Such stimuli may be exposed from the outside of the cell, by for example the use of compounds getting into contact with the cell and for example binding the cell surface or any agent on the surface of the cell, energy radiation exposure (of any wavelength), use of nuclear radiation change of conditions such as cold or heat, or agents being internalized by the cell and inducing a stimulus within the cell. Also included are stimuli which are induced by time, or internally via biochemical pathways, cellular rhythms, such as cell cycle, biochemical cycles, circadian rhythms, aging or the like. In a preferred embodiment, the stimulus is exposed to the cell by an candidate compound or composition that is contacted with the first cell. Compounds and compositions shall include second cell types (see below), small molecules, nucleic acids, proteins, such as antibodies or any derivatives of antigen binding proteins, and any combinations and/or hybrids of such compounds. Such compounds and compositions may be produced by using a second cell to be encapsulated into the compartment, such as preferably an antibody producing cell (B-cell).

Alternatively or additionally to encapsulating a second biological cell type, in some embodiments barcoded beads or particles could be used, such as DNA-barcoded one-bead-one-compound libraries. Such synthetic compounds also should provide a compound-specific barcode (and as long as there is more than one compound molecule per droplet - therefore beads instead of individually labelled compound molecules).

The term "second cell" as used herein refers to a cell secreting the polypeptide ligand or presenting the polypeptide ligand on its surface. In particular, the second cell is a cell of the B-cell lineage, preferably a plasma cell. Most preferably, the cell of the B-cell lineage is derived from a vertebrate immunized with the first cell or one or more molecules or parts thereof accessible on the surface of the first cell, for example membrane extracts. In another embodiment, the second cell is a cell expressing a polypeptide ligand derived from a library.

However, alternatively, the candidate compound or composition may be provided by using a so called "cell-free expression system", the term as used herein refers to a combination of molecules necessary for producing protein from an input RNA or DNA, such as a plasmid. The combination may comprise ribosomes, tRNAs, amino acids, including amino acyl tRNAs, RNA polymerase, ribonucleotides, and any necessary cofactors, buffering agents and salts that are required for enzymatic activity, and may include a cell lysate. Examples of cell-free expression systems include, but are not limited to, cell-free extracts of bacteria (like E. coli) or eukaryotic cells (like rabbit reticulocytes) containing transcription and translation systems required to produce mRNA (if the input is DNA) and protein. Specific examples of cell-free expression systems are the Expressway^{™} Plus expression system supplied by Invitrogen (Carlsbad, CA, USA) or the reticulocyte lysate system supplied by Roche Diagnostics (Mannheim, Germany).The scope of a cell-free expression system herein, e.g. when it is referred to "a" cell-free expression system or "one" cell-free expression system is determined by the polypeptide ligand it expresses. In other words, a cell-free expression system is a single cell-free expression system if it comprises input RNA or DNA for only one polypeptide ligand.

In a preferred embodiment of the first aspect, the candidate compound or composition is a polypeptide ligand either encapsulated directly, or expressed by a second cell or cell-free expression system has a constant region and a variable region. Therein, the variable region usually determines the binding specificity and the constant region provides the framework. Further, it is preferred that the polypeptide ligand of each second cell or cell-free expression system has the same constant region. Generally, the plurality of (micro)fluidic compartments comprises different polypeptide ligands, in particular peptide ligands with different variable regions.

In one embodiment, the polypeptide ligand is selected from the group consisting of an antibody, an antibody derivative and an antibody mimetic. The antibody, antibody derivative or antibody mimetic may be mono-specific (i.e. specific to one target molecule or part thereof accessible on the surface of a cell) or multi- specific (i.e. specific to more than one target molecule or part thereof accessible on the surface of the same or a different cell), for example bi- specific or tri-specific (see, e.g., Castoldi et al., Oncogene. 2013 Dec 12;32(50):5593-601; Castoldi et al., Protein Eng Des Sel. 2012 Oct;25(10):551-9).

The term "antibody derivative" as used herein refers to a molecule comprising at least one antibody variable domain, but not having the overall structure of an antibody such as IgA, IgD, IgE, IgG, IgM, IgY or IgW, although still being capable of binding a target molecule. Said derivatives maybe, but are not limited to functional (i.e. target binding, particularly specific target binding) antibody fragments such as Fab, Fab2, scFv, Fv, or parts thereof, or other derivatives or combinations of the immunoglobulins such as nanobodies, diabodies, minibodies, camelid single domain antibodies, single domains or Fab fragments, domains of the heavy and light chains of the variable region (such as Fd, VL, including Vlambda and Vkappa, VH, VHH) as well as mini-domains consisting of two beta- strands of an immunoglobulin domain connected by at least two structural loops. Preferably, the antibody derivative is monovalent

The term "antibody mimetic" as used herein refers to organic compounds that, like antibodies, can specifically bind antigens, but that are not structurally related to antibodies. They are usually artificial peptides or proteins with a molar mass of about 3 to 20 kDa. Non-limiting examples of antibody mimetics are affibodies, affilins, affimers, affitins, anticalins, avimers, DARPins, fynomers, Kunitz domain peptides, monobodies, Z domain of Protein A, Gamma B crystalline, ubiquitin, cystatin, Sac7D from Sulfolobus acidocaldarius, lipocalin, A domain of a membrane receptor, ankyrin repeat motive, SH3 domain of Fyn, Kunits domain of protease inhibitors, the 10th type III domain of fibronectin, synthetic heterobivalent or heteromultivalent ligands (Josan et al., Bioconjug Chem. 2011 22(7): 1270- 1278; Xu et al., PNAS 2012 109 (52) 21295-21300; Shallal et al., Bioconjug Chem. 2014 25(2) 393-405) or synthetic peptide ligands, e.g. from a (random) peptide library. Synthetic peptide ligands have non-naturally occurring amino acid sequences that function to bind a particular target molecule. Peptide ligands within the context of the present invention are generally constrained (that is, having some element of structure as, for example, the presence of amino acids which initiate a β turn or β pleated sheet, or for example, cyclized by the presence of disulfide bonded Cys residues) or unconstrained (linear) amino acid sequences of less than about 50 amino acid residues, and preferably less than about 40 amino acids residues. Of the peptide ligands less than about 40 amino acid residues, preferred are the peptide ligands of between about 10 and about 30 amino acid residues.

The term "intended to specifically bind" as used herein refers to potential binding reaction that is intentional or desired. It is not an accidental binding reaction that may occur because a second cell or cell-free expression system happens to express a polypeptide binding to a molecule or part thereof accessible on the surface of the first cell, which may occur by co-compartmentalizing of a first cell and a second cell or cell-free expression system by pure chance or accident. Intended means, for example, that the co-compartmentalizing is performed as part of a screen which is designed to identify polypeptide ligands which bind to a molecule or part thereof accessible on the surface of the first cell and/or trigger a certain, in particular a desired effect on gene expression of the first cell. In one embodiment and in particular in the embodiment in which the second cell is derived from immunization of an animal with the first cell as described herein, it is preferred that the subset of compartments referred to herein (e.g. the at least 5%, 10%, 13.5% or 20% subset) comprises one second cell or one cell-free expression system expressing a polypeptide ligand that does in fact specifically bind to a molecule or part thereof accessible on the surface of the first cell. In the embodiment described herein in which polypeptide ligands from a library not specific for the first cell are used, however, it is likely that not all polypeptide ligands expressed by the second cells or cell-free expression systems of said compartment subset specifically bind to a molecule or part thereof accessible on thesurface of the first cell. Nevertheless, they are still intended to.

The term "specifically binds" as used herein refers to a binding reaction which is determinative of the presence of the binding partner, in this case the molecule or part thereof accessible on the surface of the first cell, in a heterogeneous population of such binding partners and, in particular, cells, such as in an organism, preferably a human body. As such, the specified ligand binds to its particular target molecule and does not bind in a substantial amount to other molecules present on cells or to other molecules to which the ligand may come in contact in an organism. Generally, a ligand that "specifically binds" a target molecule has an equilibrium affinity constant greater than about 105 (e.g., 106, 107, 108, 109, 1010, 1011, and 1012 or more) mole/liter for that target molecule.

In one embodiment, regarding the target molecule or part thereof accessible on the surface of the first cell, the target molecule is a cellular receptor, more preferably a cell signaling receptor. Preferably, it is selected from the group consisting of a protein, a glycolipid or a glycoside. In a particular embodiment, the target molecule is a protein selected from the group consisting of a G-protein coupled receptor, an ion channel and a cross-membrane transporter.

The present invention makes use of a differentiation between new and old transcriptomes with regard to contacting the first cell with a certain stimulus. As such the invention provides a method using a label agent, which can be in accordance with the invention any method or compound that allows for such a differentiation. One example of the use of 4sU as a nucleoside that is incorporated into transcribed RNA and can by chemical conversion be used to induce a C to T conversion in such molecules which then can be identified by analysing sequencing information. Such methodologies are described within the herein cited prior art.

As an alternative to 4sU metabolic labelling of new RNA, also 4tU metabolic labeling is a way of detecting new RNA. Neymotin et al. (Determination of in vivo RNA kinetics using RATE-seq. RNA. 2014;20:1645-52.) used 4-thiouracil (4tU) labeling of RNA followed by RNA sequencing. The thiolated new RNA can then be coupled to biotin and selectively purified from the "old" RNA, reverse transcribed and sequenced.

In a preferred embodiment of the first aspect, the first cell is the same or of the same cell type (e.g. a tumour cell) for each compartment.

In a preferred embodiment of the invention, the candidate compound or composition is an antigen binding protein, and preferably is provided by encapsulating a second cell which expresses and releases the candidate antigen binding protein into the (micro)fluidic compartment after encapsulation in step (b).

The method of any of the preceding claim, further comprising encapsulating within the compartment a set of barcode oligonucleotides each comprising a barcode sequence unique to the set and a sequence capable of binding specifically to mRNA and/or cDNA.

The term "barcode oligonucleotide" as used herein refers to an oligonucleotide having at least one so-called variable region ("barcode sequence"), the nucleotide sequence of which is the same within the same set of oligonucleotides compared to barcode oligonucleotides of other sets used. "Variable" therein means not that the sequence of a particular oligonucleotide can change, but that there are oligonucleotides which are identical in structure and sequence with the exception of the sequence of the variable regions, i.e. the variable regions are different between oligonucleotides that are otherwise identical in structure and sequence. The length of the variable region, is preferably 1 to 50 nucleotides, more preferably 1 to 20 nucleotides and most preferably 2 to 10 nucleotides. The overall length of the "barcode oligonucleotide" is preferably 10 to 100 nucleotides, more preferably 10 to 50 nucleotides and most preferably 10 to 25 nucleotides.

In a preferred embodiment, the barcode oligonucleotide further comprises a unique molecular identifier (UMI). A "UMI" is an oligonucleotide sequence which is unique (or random with a length that makes uniqueness likely) for each oligonucleotide molecule of all oligonucleotides in a set and/or all oligonucleotides linked to a solid particle. This can improve reliability of amplification and reduce amplification noise (see, e.g. S. Islam et ah, Quantitative single-cell RNA-seq with unique molecular identifiers. Nature methods 11, 163, Feb, 2014). Also, UMIs can be used to digitally count the mRNA transcripts after sequencing. Therein, preferably, all sequenced amplificates having the same barcode sequence and the same UMI are counted as one, i.e. a single event or single mRNA transcript. To achieve uniqueness of the UMIs, their diversity 4N (N being the number of nucleotides per UMI) is preferably at least 10 times larger than the number of compartments in the subset as defined above, preferably than the number of compartments in the plurality of (micro)fluidic compartments.

The term "set of barcode oligonucleotide" as used herein refers to a plurality of barcode oligonucleotides having the same barcode sequence and preferably each a UMI unique within the set and/or within the barcode oligonucleotides linked to a solid particle. In a preferred embodiment, each set of barcode oligonucleotides is linked to one or more, preferably one solid particle, preferably a bead or a nanoparticle. If it is linked to more than one solid particle, each solid particle preferably comprises the complete set, i.e. the solid particles are preferably identical copies of each other, wherein each copy comprises the complete set. Most preferably the solid particle is a bead. A "bead" (also termed "microbead") is a uniform polymer particle with a diameter of at least 100 nm and of up to 50 µιη, preferably of 300 nm to 10 µιη, and with a surface to which nucleic acids can bind or be coupled. A round shape is not required, i.e. the term "bead" as used herein also encompasses other shapes. The beads referred to herein are usually polyethylene or polystyrene beads or beads made of gel matrices. The term "nanoparticle" used herein refers to a particle having a diameter of from about 1 to 1000 nm, preferably 1 to 100 nm. Components of the nanoparticle may include metal such as gold, silver, copper, aluminum, nickel, palladium, platinum, alloys thereof, a semiconductor material such as CdSe, CdS, InAs, InP, or core/shell structures thereof, or organic particles such as particles made from organic polymer, lipids, sugars, or other organic materials, e.g. polystyrene, latex, acrylate, or polypeptide. Such organic particles may optionally contain some inorganic material; however, the amount of inorganic material is less than 50%, less than 25%, less than 10%, less than 5%, or less than 1%.

The term "sequence capable of binding specifically to mRNA and/or cDNA thereof' as used herein refers to a sequence that is at least 80%, preferably at least 90%, more preferably at least 95% and most preferably 100% complementary to a given mRNA and/or cDNA thereof. With respect to mRNA, it is preferred that said sequence is a sequence capable of binding specifically to an mRNA 3' poly(A) tail, in particular a poly(dT) sequence or a poly(dU) sequence Such a sequence is capable of binding specifically to any mRNA and is usually 10-60 nucleotides long, preferably 15-30 and more preferably about 20 nucleotides long. For binding to a specific mRNA and/or cDNA, the sequence capable of binding specifically to mRNA and/or cDNA thereof is capable of binding to a gene-specific sequence (the gene the mRNA is transcribed from) of the mRNA or cDNA. Such a sequence is usually 10-60 nucleotides long, preferably 15-30 and more preferably 15-25 nucleotides long.

Generally, it is preferred that the sequence capable of binding specifically to mRNA and/or cDNA thereof is at the 3' end of the barcode oligonucleotide and is capable of priming a DNA polymerisation, either from an mRNA template or a cDNA template. If the sequence is capable of binding to a gene-specific sequence, it is preferred that the compartments of said subset further comprise a further oligonucleotide forming a primer pair with said sequence that is capable of binding to a gene- specific sequence, wherein the primer pair is suitable for generating a DNA amplicon from the mRNA and/or cDNA thereof. In a preferred embodiment, the sequence capable of binding specifically to mRNA and/or cDNA thereof is the same for each barcode oligonucleotide of the set.

In a **second aspect,** the invention pertains to a plurality of (micro)fluidic compartments, wherein at least 1% of said compartments form a subset in which each compartment comprises
(a) A first cell;
(b) A candidate compound or composition;
(c) A label-agent for selectively labeling synthesized mRNA in the first cell; and
(d) Optionally, a set of barcode oligonucleotides each comprising a barcode sequence unique to the set and a sequence capable of binding specifically to mRNA and/or cDNA.

In a **third aspect,** the invention pertains to a method for generating a plurality of (micro)fluidic compartments according to the second aspect, comprising the steps of:
(a) introducing into a (micro)fluidic system: (i) a fluid comprising a plurality of first cells, (ii) a fluid comprising a plurality of candidate compounds or compositions, (iii) a fluid comprising a label-agent for selectively labeling synthesized mRNA in the first cell; and (iv) a fluid comprising sets of barcode oligonucleotides, wherein the barcode oligonucleotides of each set comprises a barcode sequence unique to the set, and a sequence capable of binding specifically to mRNA and/or cDNA, and
(b) repeatedly co-compartmentalizing a first cell, a candidate compound or composition, a label-agent for selectively labeling synthesized mRNA, and a set of barcode oligonucleotides into (micro)fluidic compartments, such that the size of the subset of compartments in the plurality of compartments is at least 1%.

In further embodiments, the size of said subset of compartments, i.e. the least 1% of compartments forming a subset in which each compartment comprises the specified components, is at least 2, 3, 4, 5, 6, 7, 8, 9, 10, preferably 13.5% or even more than 20% of the total number of the plurality of compartments, wherein each larger percentage listed is preferred to the next smaller one. In a particular embodiment, the percentage is at least 5%.

Additionally, less than 25%, preferably less than 20% and more preferably less than 15% of the compartments of the plurality of (microfluidic) compartments form a "blank" subset (when it is referred above or below to a subset, the subset above and not the blank subset is meant) in which the compartments are empty. "Empty" in this respect means that a compartment does not comprise a first cell, a second cell or cell-free expression system, or a set of barcode oligonucleotides as defined above (items (i) to (iii)). A "blank" subset is a subset only comprising such empty compartments.

Each compartment of the subset of the first aspect may comprise more than one first cell, e.g. up to 2, 3, 4, 5, 6, 7, 8, 9 or 10 first cells, wherein lower upper limits are preferred to higher ones. In the most preferred embodiment of the method of the first aspect, each compartment of said subset comprises exactly one first cell.

In **a fourth aspect,** the invention pertains to a method for determining a differential gene expression of a cell, comprising the steps:
(a) Providing a first cell exposed to a stimulus, such as a candidate compound or composition, wherein the candidate compound or composition is screened for a stimulus (effect) on the first cell;
(b) Encapsulating the first cell and, optionally the compound or composition, into a (micro)fluidic compartment and, optionally, thereby bringing into contact the candidate compound or composition and the first cell;
(c) Incubating the (micro)fluidic compartment for a time sufficient to allow the first cell to change its transcriptome in response to the stimulus, such as contacting the candidate compound or composition;
(d) lysing the first cell comprised in the (micro)fluidic compartment,
(e) Determining by sequencing a transcriptome of the first cell, wherein the determining the transcriptome of the first cell comprises determining a new transcriptome of the first cell which comprises newly synthesized mRNA expressed in the first cell after encapsulation in step (b) and an old transcriptome of the first cell which comprises synthesized mRNA expressed and present before encapsulation in step (b);
(f) Providing sequencing data of a control new transcriptome (pre-identified) of one or more control cell(s) not contacted with the stimulus, such as the candidate compound or composition, wherein the first cell and the one or more control cell(s) are genetically clonal and comprise the same purposefully introduced genetic modifications;
(g) comparing the new transcriptome of first cell with the control new transcriptome wherein a change in the new transcriptome of the first cell compared to the control new transcriptome indicates an effect of the stimulus, such as a compound or composition, on the first cell.

In **an alternative fourth aspect,** the invention pertains to a method for determining a differential gene expression of a cell, comprising the steps:
(a) Providing a first cell exposed to a stimulus, such as a candidate compound or composition, wherein the candidate compound or composition is screened for an stimulus (effect) on the first cell;
(b) Encapsulating the first cell and, optionally the compound or composition, into a (micro)fluidic compartment and, optionally, thereby bringing into contact the candidate compound or composition and the first cell; wherein the (micro)fluidic compartment further comprises a label-agent for selectively labeling (newly) synthesized mRNA in the first cell;
(c) Incubating the (micro)fluidic compartment for a time sufficient to allow the first cell to change its transcriptome in response to the stimulus, such as contacting the candidate compound or composition;
(d) lysing the first cell comprised in the (micro)fluidic compartment,
(e) Determining by sequencing the transcriptome of the first cell, wherein the transcriptome of the first cell is composed of a new transcriptome comprising newly synthesized mRNA detectable by the label-agent after encapsulation in step (b), and an old transcriptome comprising previously synthesized mRNA detectable by the absence of the label-agent before encapsulation in step (b);
(f) Providing sequencing data of a control new transcriptome (pre-identified) of one or more control cell(s) not contacted with the stimulus, such as the candidate compound or composition, wherein the first cell and the one or more control cell(s) are genetically clonal and comprise the same purposefully introduced genetic modifications;
(g) comparing the new transcriptome of first cell with the control new transcriptome wherein a change in the new transcriptome of the first cell compared to the control new transcriptome indicates an effect of the stimulus, such as a compound or composition, on the first cell.

The invention is also described according to the following itemized embodiments, which should be read and understood in context of the above definitions and explanations, and are exemplified by the specific non-limiting examples provided by the present invention.
Item 1. A method for screening in a (micro)fluidic system candidate compounds or compositions for an effect on a cell, the method comprising the steps of
   (a) Providing a candidate compound or composition and a first cell, wherein the candidate compound or composition is screened for an effect on the first cell;
   (b) Encapsulating a candidate compound or composition together with a first cell into a (micro)fluidic compartment and thereby bringing into contact the candidate compound or composition and the first cell;
   (c) Incubating the (micro)fluidic compartment for a time sufficient to allow the first cell to change its transcriptome in response to contacting the candidate compound or composition;
   (d) Determining by sequencing a transcriptome of the first cell, wherein the determining the transcriptome of the first cell comprises determining a new transcriptome of the first cell which comprises newly synthesized mRNA expressed in the first cell after encapsulation in step (b) and an old transcriptome of the first cell which comprises synthesized mRNA expressed and present before encapsulation in step (b);
   (e) Wherein a change of mRNA expression in the new transcriptome compared to the old transcriptome in the first cell indicates an effect of the candidate compound or composition on the first cell.
Item 2. The method of item 1, wherein in step (b) the (micro)fluidic compartment further comprises a label-agent for selectively labeling synthesized mRNA in the first cell; and wherein in step (d) the new transcriptome of the first cell comprises newly synthesized mRNA detectable by the label-agent after encapsulation in step (b) and wherein the old transcriptome of the first cell comprises synthesized mRNA detectable by the absence of the label-agent before encapsulation in step (b).
Item 3. The method of item 2, wherein the label-agent is a nucleotide analog which can be incorporated into transcribed mRNA, and thereby label newly synthesized mRNA.
Item 4. The method of item 2 or 3, wherein the label-agent is a protein binding epitope, such as an antibody or receptor-ligand binding epitope, a biotin-streptavidin protein, 4sU, or a derivative or analog thereof.
Item 5. The method of item 1, wherein determining the new transcriptome of the first cell and determining the old transcriptome of the first cell comprises a step of determining and comparing a fraction of spliced mRNA and unspliced mRNA in the sequenced transcriptome of the first cell.
Item 6. The method of any one of items 1 to 5, wherein step (d) further comprises a comparison of the new transcriptome of first cell with a pre-identified new transcriptome of a one or more control cell(s) not contacted with any candidate compound or composition, and wherein a change in the new transcriptome of the first cell compared to the new transcriptome of the one or more control cell(s) indicates an effect of the compound or composition on the first cell; wherein the first cell and the one or more control cell(s) are genetically clonal and comprise the same purposefully introduced genetic modifications.
Item 7. The method of item 6, wherein the candidate compound or composition has an effect on the first cell if the number of differentially expressed mRNA in the new transcriptome of the first cell compared to the new transcriptome of the one or more control cell(s) divided by the number of total expressed mRNA species (expressed genes) in the old transcriptome of the first cell is higher than a predetermined threshold P.
Item 8. The method of item 5 or 6, wherein the determination of an old or new transcriptome of the more than one control cells involves taking the average of the transcriptomes of the more than one control cells.
Item 9. The method of any of the preceding items, wherein the candidate compound or composition is a candidate antigen binding protein.
Item 10. The method of item 9, wherein the candidate antigen binding protein is provided by encapsulating a second cell which expresses and releases and/or presents on its surface the candidate antigen binding protein into the (micro)fluidic compartment after encapsulation in step (b).
Item 11. The method of any of the preceding item, further comprising encapsulating within the compartment a set of barcode oligonucleotides each comprising a barcode sequence unique to the set (and unique to one compartment of a plurality of compartments) and a sequence capable of binding specifically to mRNA and/or cDNA.
Item 12. The method of item 11, wherein the set of barcode oligonucleotides is linked to a micro or nanoparticle, such as a bead.
Item 13. The method of item 11 or 12, wherein said sequence capable of binding specifically to mRNA and/or cDNA is a sequence capable of binding specifically to an mRNA 3' poly(A) tail or to a gene-specific sequence.
Item 14. The method of any of the preceding items, wherein the candidate compound or composition has a candidate effect on a cell surface receptor expressed on the cell surface of the first cell.
Item 15. A plurality of (micro)fluidic compartments, wherein at least 1% of said compartments form a subset in which each compartment comprises
   (a) A first cell;
   (b) A candidate compound or composition;
   (c) A label-agent for selectively labeling synthesized mRNA in the first cell; and
   (d) Optionally, a set of barcode oligonucleotides each comprising a barcode sequence unique to the set and a sequence capable of binding specifically to mRNA and/or cDNA.
Item 16. A method for generating a plurality of (micro)fluidic compartments according to item 13, comprising the steps of:
   (a) introducing into a (micro)fluidic system: (i) a fluid comprising a plurality of first cells, (ii) a fluid comprising a plurality of candidate compounds or compositions, (iii) a fluid comprising a label-agent for selectively labeling synthesized mRNA in the first cell; and (iv) a fluid comprising sets of barcode oligonucleotides, wherein the barcode oligonucleotides of each set comprises a barcode sequence unique to the set, and a sequence capable of binding specifically to mRNA and/or cDNA, and
   (b) repeatedly co-compartmentalizing a first cell, a candidate compound or composition, a label-agent for selectively labeling synthesized mRNA, and a set of barcode oligonucleotides into (micro)fluidic compartments, such that the size of the subset of compartments in the plurality of compartments is at least 1%.
Item 17. A method for determining a differential gene expression of a cell, comprising the steps:
   (a) Providing a first cell exposed to a stimulus, such as a candidate compound or composition, wherein the candidate compound or composition is screened for a stimulus (effect) on the first cell;
   (b) Encapsulating the first cell and, optionally the compound or composition, into a (micro)fluidic compartment and, optionally, thereby bringing into contact the candidate compound or composition and the first cell;
   (c) Incubating the (micro)fluidic compartment for a time sufficient to allow the first cell to change its transcriptome in response to the stimulus, such as contacting the candidate compound or composition;
   (d) lysing the first cell comprised in the (micro)fluidic compartment,
   (e) Determining by sequencing a transcriptome of the first cell, wherein the determining the transcriptome of the first cell comprises determining a new transcriptome of the first cell which comprises newly synthesized mRNA expressed in the first cell after encapsulation in step (b) and an old transcriptome of the first cell which comprises synthesized mRNA expressed and present before encapsulation in step (b);
   (f) Providing sequencing data of a control new transcriptome (pre-identified) of one or more control cell(s) not contacted with the stimulus, such as the candidate compound or composition, wherein the first cell and the one or more control cell(s) are genetically clonal and comprise the same purposefully introduced genetic modifications;
   (g) comparing the new transcriptome of first cell with the control new transcriptome wherein a change in the new transcriptome of the first cell compared to the control new transcriptome indicates an effect of the stimulus, such as a compound or composition, on the first cell.
Item 18. The method of item 17, wherein in step (b) the (micro)fluidic compartment further comprises a label-agent for selectively labeling synthesized mRNA in the first cell; and wherein in step (d) the new transcriptome of the first cell comprises newly synthesized mRNA detectable by the label-agent after encapsulation in step (b) and wherein the old transcriptome of the first cell comprises synthesized mRNA detectable by the absence of the label-agent before encapsulation in step (b).
Item 19. The method of item 18, wherein the label-agent is a nucleotide analog which can be incorporated into transcribed mRNA, and thereby label newly synthesized mRNA.
Item 20. The method of item 18 or 19, wherein the label-agent is 4sU, or a derivative or analog thereof.
Item 21. The method of item 17, wherein determining the new transcriptome of the first cell and determining the old transcriptome of the first cell comprises a step of determining and comparing a fraction of spliced mRNA and unspliced mRNA in the sequenced transcriptome of the first cell.

The terms "of the [present] invention", "in accordance with the invention", "according to the invention" and the like, as used herein are intended to refer to all aspects and embodiments of the invention described and/or claimed herein.

As used herein, the term "comprising" is to be construed as encompassing both "including" and "consisting of', both meanings being specifically intended, and hence individually disclosed embodiments in accordance with the present invention. Where used herein, "and/or" is to be taken as specific disclosure of each of the two specified features or components with or without the other. For example, "A and/or B" is to be taken as specific disclosure of each of (i) A, (ii) B and (iii) A and B, just as if each is set out individually herein. In the context of the present invention, the terms "about" and "approximately" denote an interval of accuracy that the person skilled in the art will understand to still ensure the technical effect of the feature in question. The term typically indicates deviation from the indicated numerical value by ±20%, ±15%, ±10%, and for example ±5%. As will be appreciated by the person of ordinary skill, the specific such deviation for a numerical value for a given technical effect will depend on the nature of the technical effect. For example, a natural or biological technical effect may generally have a larger such deviation than one for a man-made or engineering technical effect. As will be appreciated by the person of ordinary skill, the specific such deviation for a numerical value for a given technical effect will depend on the nature of the technical effect. For example, a natural or biological technical effect may generally have a larger such deviation than one for a man-made or engineering technical effect. Where an indefinite or definite article is used when referring to a singular noun, e.g. "a", "an" or "the", this includes a plural of that noun unless something else is specifically stated.

It is to be understood that application of the teachings of the present invention to a specific problem or environment, and the inclusion of variations of the present invention or additional features thereto (such as further aspects and embodiments), will be within the capabilities of one having ordinary skill in the art in light of the teachings contained herein.

Unless context dictates otherwise, the descriptions and definitions of the features set out above are not limited to any particular aspect or embodiment of the invention and apply equally to all aspects and embodiments which are described.

All references, patents, and publications cited herein are hereby incorporated by reference in their entirety.

### BRIEF DESCRIPTION OF THE FIGURES

The figures show:
**Figure 1****: Workflow overview (A)** Target cell, antibody-secreting cell and Drop-seq beads are co-encapsulated into water in oil droplets. Cells are lysed in droplets and mRNA hybridizes to Drop-seq beads. There are two ways to improve the resolution of the transcriptomic signature of the target cells upon stimulation: **(B)** In the presence of the metabolic labeling reagent, newly synthesized mRNA incorporates 4sU. After the mRNA capturing on beads, the 4sU is chemically converted to a cytosine analog which leads to an increased amount of C-T conversions in newly transcribed mRNAs in comparison to old mRNAs in the transcriptomic sequencing results. **(C)** Without metabolic labeling reagent, unspliced and spliced mRNA, which are detected in every scRNAseq, can be used to approximate the new mRNA (unspliced mRNA) and old mRNA (spliced mRNA).

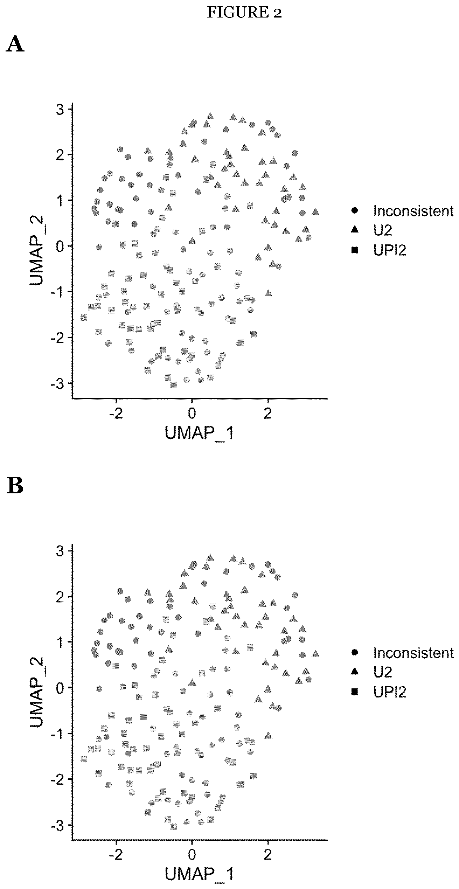

**Figure 2****:** Dimensional reduction upon unsupervised clustering of gene expression data of Jurkat cells activated for two hours with PMA and Ionomycin (UPI2) or treated with supernatant of H25 hybridoma cell line, expressing non-functional antibodies (U2H). **A.** Dimensional reduction using old mRNA. **B.** Dimensional reduction using newly-synthesized mRNA after treatment.

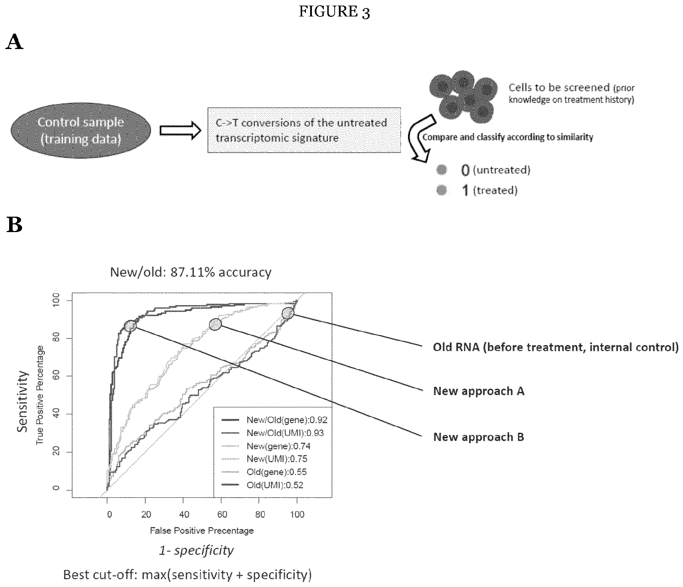

**Figure 3****:** Comparison of different classification methods to assign treated and untreated cells. **(A)** First, C-T conversions of the untreated transcriptomic signatures (media control) are subtracted from all samples. Subsequently, data from treated (Jurkat cells treated with PMA, ionomycin, and 4sU) and untreated (Jurkat cells treated with 4sU and non-functional antibody) samples are combined into a training data set, to test whether the classification method can assign the cells correctly. **(B)** ROC curve comparing three different classification approaches. 1. Using only old mRNA, 2. Approach A using only new mRNA, 3. Approach B using the ratio of new and old mRNA.

### EXAMPLES

Certain aspects and embodiments of the invention will now be illustrated by way of example and with reference to the description, figures and tables set out herein. Such examples of the methods, uses and other aspects of the present invention are representative only, and should not be taken to limit the scope of the present invention to only such representative examples.

The examples show:
The inventors implemented exemplary a chemical 4sU based strategy for labelling and determining newly synthesized mRNA(newtranscriptome) within the prior art derived workflow and analysed the unsupervised clustering of cells according to (known) treatment conditions. The details of the approach is shown in Figure 1. Clustering was used for data analysis as done before and worked well for groups of cells having been exposed to the same treatment, and supports the applicability of the method of the invention (see Figure 2). A general strategy for data analysis is shown below.

However, the approach still was not satisfactory in identifying individual outliers. The inventors then completely abolished the clustering-based analysis and rather established two new approaches. They both make use of a reference/training data set (C->T conversions observed in the untreated cells) and then compare every single cell of the test data set to this signature (Slide 8). C->T conversions that are not observed in the untreated control are summed up in terms of the number of corresponding/affected genes (= n) and a threshold for n is defined above which a cell is considered as an outlier that rather represents a "treated" phenotype (of whatever kind, so note that this is still entirely target agnostic). This approach (New Method A) is already powerful, but it still shows a relatively high false discovery rate.

The inventors hence refined the approach (New method B) in the way that they divided n by the number of genes that was detected in the old mRNA data set of the same cell (note that upon labelling one will still find all pre-existing mRNA without C->T conversions). This approach basically compares the new mRNA of every single cell (synthesized after treatment or e.g. exposure to specific antibodies) to the set of pre-existing mRNA, synthesized before treatment. It is therefore much less affected by the different expression levels of genes across different cells and in turn enables a much higher resolution. In fact, new method B allowed to classify individual cells correctly as either treated or untreated with an overall efficiency of 87.11% (Figure 3). In conclusion, this approach overcomes the problems in implementing WO2017121832A1 and we therefore seek patent protection.

An exemplary procedure for data analysis is provided here:

Data analysis workflow (as used for the experiment shown in Figure 3):
In a model experiment, Jurkat cells were treated outside of (micro)fluidic droplets with: (i) PMA (Phorbol myristate acetate, an activator of the NF-kB pathway) and ionomycine (= positive control), (ii) Media only (= media control); (iii) A control antibody known to have no functional effect on Jurkat cells (= negative control).

In all cases, 4sU was added for labeling of new mRNA, and different hashtag antibodies (antibodies linked to an oligonucleotide barcode with a polyA end - allowing to encode a treatment condition of a particular cell in an RNA sequencing library) were added to barcode the treatment condition (hashtag antibody 1 for the PMA and Ionomycin treated sample, hashtag antibody 2 for the untreated sample and hashtag 3 for the sample treated with a control antibody). Then the cells were encapsulated into droplets at the single cell level together with DropSeq beads to enable single cell RNA sequencing. The experimental workflow was similar to that shown in Figure 1b. However only one cell type (Jurkat cells) was encapsulated, being pre-treated prior to encapsulation, mimicking the effect of the second cell type. After sequencing the RNA library, the data set was processed as follows:
- Sequencing data was first demultiplexed according to the hash-tag labelling (read 2, allowing 3 mismatches). This allowed to split the data into treated and untreated samples. Next, read 1 was used to obtain the different DropSeq barcodes, enabling to demultiplex the sample-specific transcriptomic data down to the single cell level.
- Sample reads were filtered based on quality control and then mapped to the human reference genome to obtain information on T->C conversion.
- T->C conversions observed in the media control were removed from the data sets of the positive and negative control.
- The remaining T->C conversions were used to label mapped reads as new mRNAs reads (with T->C) and old mRNAs reads (without T->C)
- For each cell, the total number of genes and UMIs showing newly synthesized mRNA and old mRNA was calculated. Then, the ratio of these values (new/old) was determined.
- The AUC was calculated to identify the best cutoff value (in terms of new/old ratio) for classifying cells as either treated (positive control) or untreated (negative control).
- Using this cutoff value, cells from positive and negative control samples were classified as treated or untreated at the single cell level in an unsupervised manner. Then the classification (= predicted treatment) was compared to the hashtag present in the corresponding droplet (= real treatment) resulting in an 87.11% accuracy.

## Claims

1. **A method for screening in a fluidic, such as a microfluidic, system candidate compounds or compositions for an effect on a cell,** the method comprising the steps of
(a) Providing a candidate compound or composition and a first cell, wherein the candidate compound or composition is screened for an effect on the first cell;
(b) Encapsulating a candidate compound or composition together with a first cell into a fluidic, such as a microfluidic, compartment and thereby bringing into contact the candidate compound or composition and the first cell;
(c) Incubating the fluidic, such as a microfluidic, compartment for a time sufficient to allow the first cell to change its transcriptome in response to contacting the candidate compound or composition;
(d) Determining by sequencing a transcriptome of the first cell, wherein the determining the transcriptome of the first cell comprises determining a new transcriptome of the first cell which comprises newly synthesized mRNA expressed in the first cell after a predetermined point in time, such as the encapsulation in step (b), and an old transcriptome of the first cell which comprises synthesized mRNA expressed and present before the predetermined point in time, such as the encapsulation in step (b);
(e) Wherein a change of mRNA expression in the new transcriptome compared to the old transcriptome in the first cell indicates an effect of the candidate compound or composition on the first cell.

2. The method of claim 1, wherein in step (b) the fluidic, such as a microfluidic, compartment further comprises a label-agent for selectively labeling synthesized mRNA in the first cell; and wherein in step (d) the new transcriptome of the first cell comprises newly synthesized mRNA detectable by the label-agent after encapsulation in step (b) and wherein the old transcriptome of the first cell comprises synthesized mRNA detectable by the absence of the label-agent before encapsulation in step (b).

3. The method of claim 2, wherein the label-agent is a nucleotide analog which can be incorporated into transcribed mRNA, and thereby label newly synthesized mRNA.

4. The method of claim 2 or 3, wherein the label-agent is a protein binding epitope, such as an antibody or receptor-ligand binding epitope, a biotin-streptavidin protein, 4sU, or a derivative or analog thereof.

5. The method of claim 1, wherein determining the new transcriptome of the first cell and determining the old transcriptome of the first cell comprises a step of determining and comparing a fraction of spliced mRNA and unspliced mRNA in the sequenced transcriptome of the first cell.

6. The method of any one of claims 1 to 5, wherein step (d) further comprises a comparison of the new transcriptome of first cell with a pre-identified new transcriptome of a one or more control cell(s) not contacted with any candidate compound or composition, and wherein a change in the new transcriptome of the first cell compared to the new transcriptome of the one or more control cell(s) indicates an effect of the compound or composition on the first cell; wherein the first cell and the one or more control cell(s) are genetically clonal and comprise the same purposefully introduced genetic modifications.

7. The method of claim 6, wherein the candidate compound or composition has an effect on the first cell if the number of differentially expressed mRNA in the new transcriptome of the first cell compared to the new transcriptome of the one or more control cell(s) divided by the number of total expressed mRNA species (expressed genes) in the old transcriptome of the first cell is higher than a predetermined threshold P.

8. The method of any of the preceding claims, wherein the candidate compound or composition is a candidate antigen binding protein.

9. The method of claim 9, wherein the candidate antigen binding protein is provided by encapsulating a second cell which expresses and releases and/or presents on its surface the candidate antigen binding protein into the fluidic, such as a microfluidic, compartment after encapsulation in step (b).

10. **A plurality of fluidic, such as a microfluidic, compartments,** wherein at least 1% of said compartments form a subset in which each compartment comprises
(a) A first cell;
(b) A candidate compound or composition;
(c) A label-agent for selectively labeling synthesized mRNA in the first cell; and
(d) Optionally, a set of barcode oligonucleotides each comprising a barcode sequence unique to the set and a sequence capable of binding specifically to mRNA and/or cDNA.

11. **A method for generating a plurality of fluidic, such as a microfluidic, compartments according to claim 13**, comprising the steps of:
(a) introducing into a fluidic, such as a microfluidic, system: (i) a fluid comprising a plurality of first cells, (ii) a fluid comprising a plurality of candidate compounds or compositions, (iii) a fluid comprising a label-agent for selectively labeling synthesized mRNA in the first cell; and (iv) a fluid comprising sets of barcode oligonucleotides, wherein the barcode oligonucleotides of each set comprises a barcode sequence unique to the set, and a sequence capable of binding specifically to mRNA and/or cDNA, and
(b) repeatedly co-compartmentalizing a first cell, a candidate compound or composition, a label-agent for selectively labeling synthesized mRNA, and a set of barcode oligonucleotides into fluidic, such as a microfluidic, compartments, such that the size of the subset of compartments in the plurality of compartments is at least 1%.

12. **A method for determining a differential gene expression of a cell,** comprising the steps:
(a) Providing a first cell exposed to a stimulus, such as a candidate compound or composition, wherein the candidate compound or composition is screened for a stimulus (effect) on the first cell;
(b) Encapsulating the first cell and, optionally the compound or composition, into a fluidic, such as a microfluidic, compartment and, optionally, thereby bringing into contact the candidate compound or composition and the first cell;
(c) Incubating the fluidic, such as a microfluidic, compartment for a time sufficient to allow the first cell to change its transcriptome in response to the stimulus, such as contacting the candidate compound or composition;
(d) lysing the first cell comprised in the fluidic, such as a microfluidic, compartment,
(e) Determining by sequencing a transcriptome of the first cell, wherein the determining the transcriptome of the first cell comprises determining a new transcriptome of the first cell which comprises newly synthesized mRNA expressed in the first cell after encapsulation in step (b) and an old transcriptome of the first cell which comprises synthesized mRNA expressed and present before encapsulation in step (b);
(f) Providing sequencing data of a control new transcriptome (pre-identified) of one or more control cell(s) not contacted with the stimulus, such as the candidate compound or composition, wherein the first cell and the one or more control cell(s) are genetically clonal and comprise the same purposefully introduced genetic modifications;
(g) comparing the new transcriptome of first cell with the control new transcriptome wherein a change in the new transcriptome of the first cell compared to the control new transcriptome indicates an effect of the stimulus, such as a compound or composition, on the first cell.

13. The method of claim 12, wherein in step (b) the fluidic, such as a microfluidic, compartment further comprises a label-agent for selectively labeling synthesized mRNA in the first cell; and wherein in step (d) the new transcriptome of the first cell comprises newly synthesized mRNA detectable by the label-agent after encapsulation in step (b) and wherein the old transcriptome of the first cell comprises synthesized mRNA detectable by the absence of the label-agent before encapsulation in step (b).

14. The method of claim 13, wherein the label-agent is a nucleotide analog which can be incorporated into transcribed mRNA, and thereby label newly synthesized mRNA, such as 4sU.

15. The method of claim 12, wherein determining the new transcriptome of the first cell and determining the old transcriptome of the first cell comprises a step of determining and comparing a fraction of spliced mRNA and unspliced mRNA in the sequenced transcriptome of the first cell.
